(19) Europäisches Patentamt
European Patent Office
Office européen des brevets

(11) Numéro de publication: **0 304 648 B1**

(12) **FASCICULE DE BREVET EUROPEEN**

(45) Date de publication de fascicule du brevet: **23.09.92**

(51) Int. Cl.[5]: **C07D 239/48**, C07D 239/50

(21) Numéro de dépôt: **88112023.2**

(22) Date de dépôt: **26.07.88**

# (54) Procédé de préparation de pipéridino-6 diamino-2,4 pyrimidine oxyde-3 et composés.

(30) Priorité: **31.07.87 LU 86960**

(43) Date de publication de la demande:
**01.03.89 Bulletin 89/09**

(45) Mention de la délivrance du brevet:
**23.09.92 Bulletin 92/39**

(84) Etats contractants désignés:
**AT BE CH DE ES FR GB GR IT LI NL SE**

(56) Documents cités:
**AU-B- 519 993**
**US-A- 3 382 247**

(73) Titulaire: **L'OREAL**
**14, Rue Royale**
**F-75008 Paris(FR)**

(72) Inventeur: **Maignan, Jean**
**8, Rue Halévy**
**F-93290 Tremblay les Gonesse(FR)**
Inventeur: **Restle, Serge**
**140 Rue Anatole France**
**F-93601 Aulnay sous Bois(FR)**
Inventeur: **Lang, Gérard**
**44, Avenue Lacour**
**F-95210 Saint Gratien(FR)**

(74) Mandataire: **Casalonga, Axel et al**
**BUREAU D.A. CASALONGA - JOSSE Morassistrasse 8**
**W-8000 München 5(DE)**

EP 0 304 648 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à la préparation de pipéridino-6 diamino-2,4 pyrimidine oxyde-3 plus connu sous la dénomination internationale générique de "Minoxidil".

Le "Minoxidil" est connu en lui-même pour ses propriétés antihypertensives ainsi que pour son application dans le traitement de l'alopécie et de la pelade.

On connaît déjà plusieurs procédés de préparation de la pipéridino-6 diamino-2,4 pyrimidine oxyde-3. C'est ainsi que le brevet US-3.382.247 décrit un procédé consistant à préparer dans un première étape la chloro-6 diamino-2,4 pyrimidine à partir de l'hydroxy-6 diamino-2,4 pyrimidine par traitement à l'oxychlorure de phosphore. Par réaction d'un phénate sur la chloro-6 diamino 2,4-pyrimidine, on obtient la phénoxy-6 diamino-2,4 pyrimidine. Cette dernière est oxydée par l'acide métachloroperbenzoïque (MCPA) en N-oxyde-3 correspondant. Après réaction avec la pipéridine, on isole le "Minoxidil".

Le brevet GB-A-2.032.434 décrit un procédé de préparation du "Minoxidil" consistant à préparer dans une première étape le dérivé O-tosylé de l'hydroxy-6 diamino-2,4 pyrimidine, à oxyder ce dernier en un oxyde correspondant par action de l'acide métachloroperbenzoïque et enfin à traiter ce tosylate N-oxyde par la pipéridine.

Selon le brevet US-A-3.910.928, le noyau pyrimidine est synthétisé à partir du cyanimino-3 propionitrile.

Dans les deux premières synthèses, les fonctions amine dans les positions 2 et 4 de la chloro ou de l'hydroxydiaminopyrimidine restent libres.

La demanderesse a découvert, ce qui fait l'objet de l'invention, un nouveau procédé de préparation de pipéridino-6 diamino-2,4 pyrimidine oxyde-3 ou "Minoxidil" suivant un procédé simple et peu onéreux par rapport aux procédés préconisés dans l'état de la technique.

L'invention a donc pour objet un nouveau procédé de préparation du "Minoxidil".

Un autre objet de l'invention est constitué par les produits nouveaux préparés au cours de ce procédé.

D'autres objets apparaîtront à la lecture de la description et des exemples qui suivent.

Le procédé de préparation de la pipéridino-6 diamino-2,4 pyrimidine oxyde-3, conforme à l'invention, est essentiellement caractérisé par le fait que l'on protège, dans une première étape, au moins l'une des deux fonctions amine du composé de formule (1) :

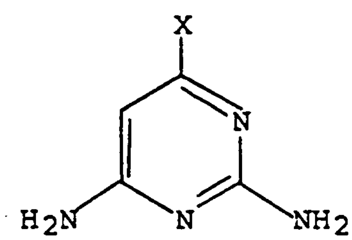

(1)

dans laquelle X désigne halogène ou un groupement OH par une réaction d'addition d'un isocyanate de formule :

R - N = C = O

dans laquelle R désigne un groupement alkyle ayant 1 à 6 atomes de carbone sur l'un des groupements amine du composé de formule (1), que l'on oxyde le dérivé d'urée résultant pour transformer le composé en N-oxyde correspondant, le N-oxyde étant ensuite amené à réagir avec la pipéridine puis soumis à une réaction d'élimination du (des) groupement(s) protecteur(s) par traitement avec une base organique ou inorganique.

Le procédé conforme à l'invention peut en particulier être illustré par les schémas réactionnels ci-après.

## SCHEMA A

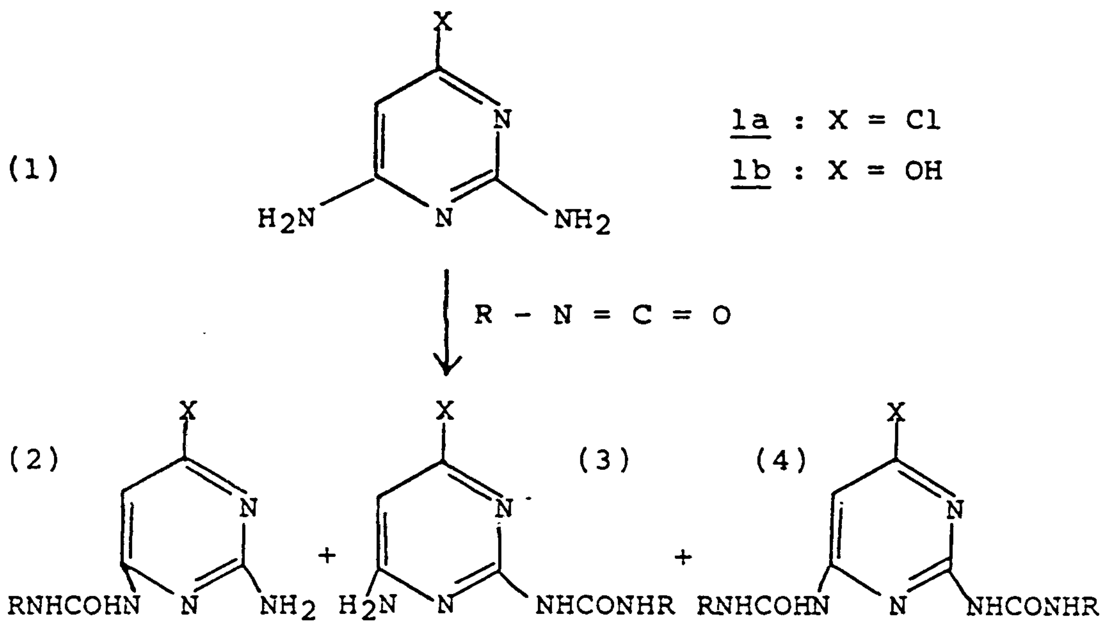


(1)

1a : X = Cl

1b : X = OH

(2) (3) (4)

2a : X = Cl    3a : X = Cl    4a : X = Cl

2b : X = OH    3b : X = OH    4b : X = OH

SCHEMA B

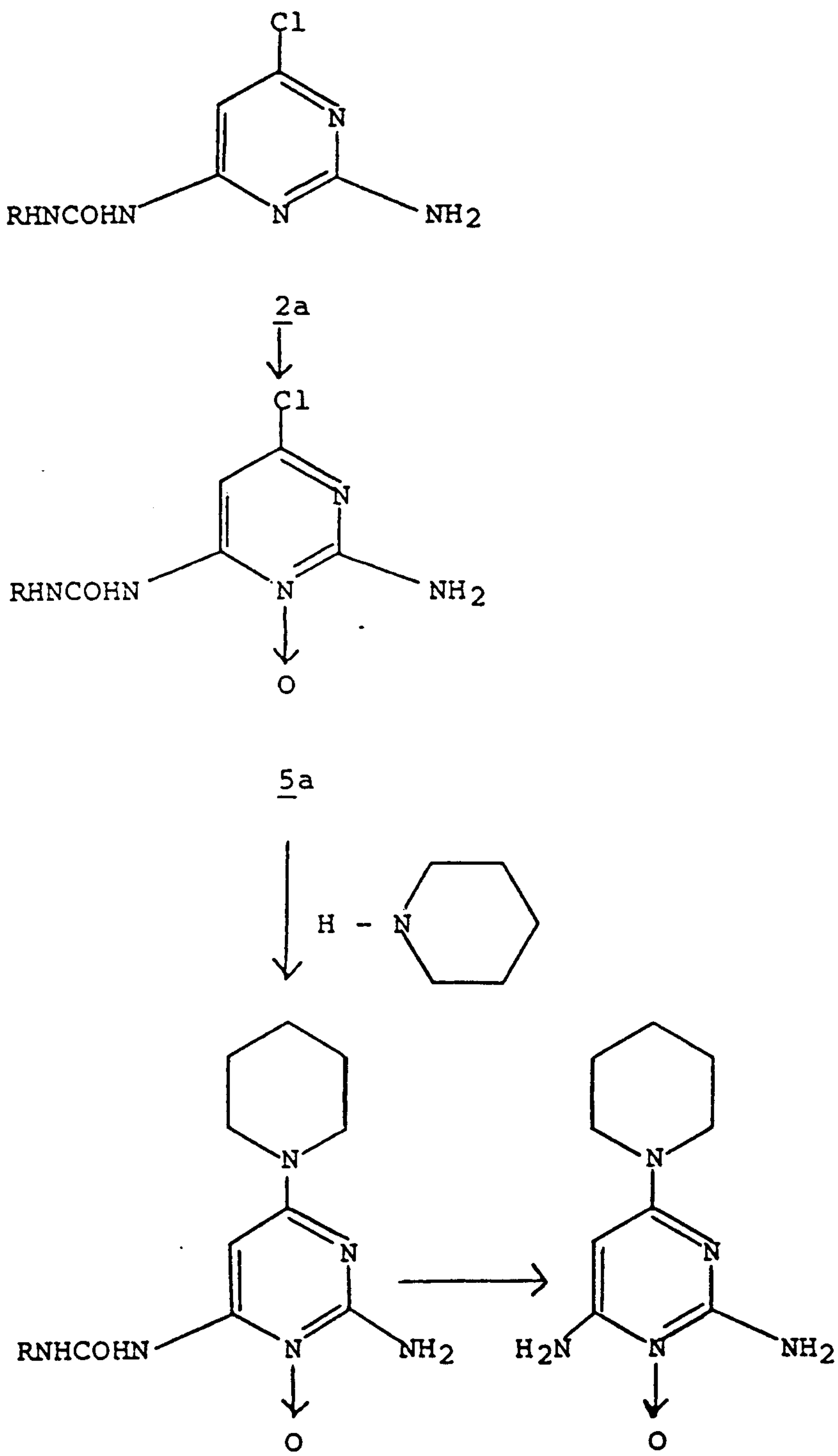

2a

5a

6a

7

SCHEMA C

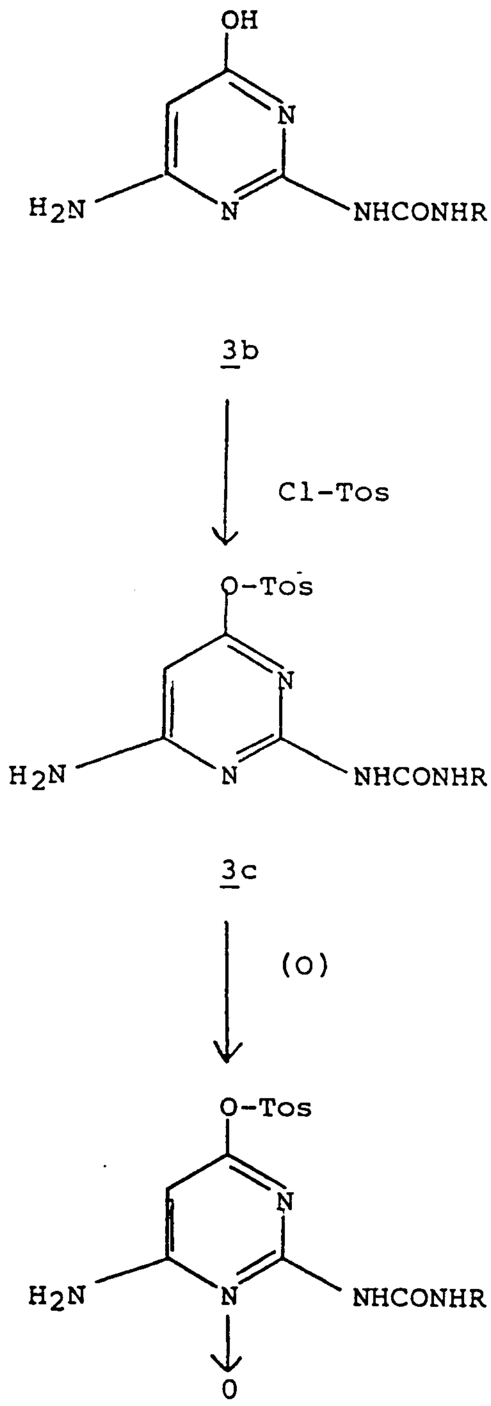


3b

3c

5c

5c

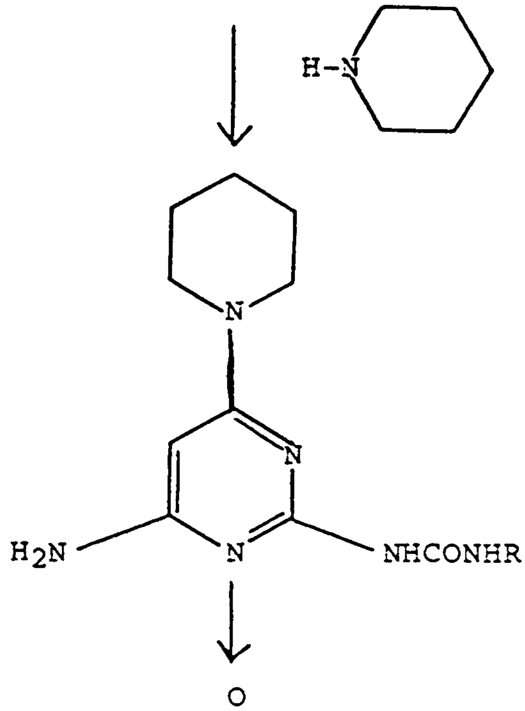

6c

7

SCHEMA A

Dans la première étape du procédé conforme à l'invention, on obtient un mélange de mono-urées de formule 2a et 3a, en partant du composé 1a, contenant suivant le temps de mise en réaction et le nombre d'équivalents d'isocyanate, une quantité plus ou moins importante de di-urées de formule 4a.

Il est possible d'orienter de façon sélective la réaction vers la formation de l'urée 2a ou 3b en utilisant des solvants aprotiques tels que plus particulièrement le diméthylsulfoxyde (DMSO), le diméthylformamide (DMF) ou la N-méthyl pyrrolidone. Il se forme l'urée 2a ou 3b suivant que le produit de départ est respectivement le produit chloré 1a ou hydroxylé 1b.

Il est possible d'éliminer facilement le peu de diurée 4a ou 4b formée, par filtration à température ambiante des mélanges réactionnels, du fait de la grande différence de solubilité dans le milieu des mono-urées ou des di-urées.

Cette première étape est particulièrement intéressante car elle met en oeuvre des produits commerciaux et facilement accessibles.

Des résultats particulièrement intéressants, notamment au niveau du rendement de la réaction, sont obtenus en utilisant le butyl-isocyanate.

En utilisant un solvant de réaction tel que le tétrahydrofuranne dans la première étape, on obtient de façon préférentielle l'urée 3a, en partant du composé 1a, en particulier lorsqu'on utilise l'isocyanate de butyle.

SCHEMA B

Au cours de la deuxième étape, l'oxydation s'effectue de façon préférentielle sur la mono-urée de

formule 2a dont la fonction amino en position 4 est bloquée. Cette urée est plus facilement oxydable en position 3. On peut opérer en particulier en phase hétérogène dans un mélange d'un solvant chloré comme par exemple le dichlorométhane en présence d'environ 5 à 30% d'acide formique et de préférence 10%, en ajoutant un excès d'eau oxygénée de 2 à 5 équivalents par rapport au composé de départ 2a et de préférence 2,5 équivalents. On obtient ainsi une transformation quantitative de l'urée 2a en N-oxyde-3 (5a) correspondant.On obtient également de très bons résultats en utilisant le dioxanne à la place du dichlorométhane.

La réaction est conduite à une température comprise entre 0 et 70 °C, et de préférence entre 35 et 65 °C.

La demanderesse a constaté qu'en opérant dans ces conditions, on ne détecte, par les moyens chromatographiques habituels, aucun dérivé secondaire ni aucun dérivé de dégradation dans des quantités notables.

SCHEMA C

Une autre variant de l'étape 2 conforme à l'invention consiste à transformer la mono-urée 3b hydroxylée en tosylate correspondant 3c suivant des méthodes connues; le composé 3c étant ensuite oxydé en un N-oxyde-3 (5c). La mono-urée 3b étant obtenue de façon préférentielle par action d'un isocyanate sur le produit de départ 1b solubilisé dans la N-méthyl pyrrolidone.

Il est à noter que le tosylate ou le benzène sulfonate peut être synthétisé, dans une première étape, à partir du composé (1b). Dans un seconde étape, ce tosylate ou benzène sulfonate est mis en réaction avec un isocyanate et on obtient alors des sulfonates de mono-urées (2b) et/ou (3b).

Dans une étape ultérieure, les composés 5a ou 5c sont amenés à réagir avec le pipéridine utilisée de préférence en excès et sont transformés respectivement en mono-urée de formule 6a ou 6c. Les rendements de transformation sont également dans ce cas particulièrement intéressants et quantitatifs.

Enfin, dans une dernière étape, on procède à l'élimination du groupement protecteur urée par traitement avec une base forte minérale ou organique telles que plus particulièrement la potasse, la soude, le méthylate de sodium, mais de préférence la potasse.

Ce procédé est particulièrement intéressant dans la mesure où il peut également s'appliquer au mélange des composés 2a, 3a, et 4a.

Le procédé permet de transformer le composé de formule (1) en une mono-urée par une réaction d'addition simple d'une fonction amine sur un isocyanate.

Les mono-urées ainsi obtenues sont beaucoup plus solubles dans les solvants habituels que les composés de départ et se prêtent particulièrement bien aux réactions d'oxydation pour obtenir les N-oxyde-3 correspondants.

Le fait d'utiliser les mono-urées conduit en particulier à des oxydations sélectives.

La réaction particulièrement préférée consiste à passer par l'intermédiaire de la chloro-6 diamino-2,4 pyrimidine qui met en oeuvre au niveau de l'oxydation l'eau oxygénée qui est l'oxydant préféré. L'utilisation de l'eau oxygénée présente également l'avantage de réduire les coûts et d'éviter les problèmes d'élimination de l'acide métachloroperbenzoïque.

Les composés intermédiaires chlorés en position 6 sous forme de N-oxyde répondant à la formule :

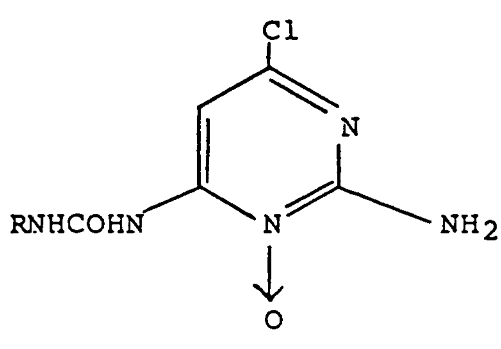

sont particulièrement intéressants du fait de leur solubilité pour le traitement par la pipéridine.

La transformation à la dernière étape de l'urée en amine correspondante est une réaction simple puisqu'elle est réalisée par action d'une base minérale ou organique.

Les urées intermédiaires, préparées au cours du procédé conforme à l'invention, sont des produits

nouveaux et constituent des objets de l'invention.

Ces composés répondent à la formule :

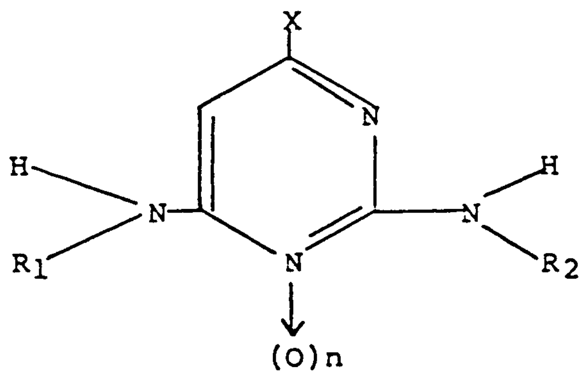

dans laquelle :

X désigne OH, Cl, O-tosyl ou O-benzène sulfonyl ou un groupement pipéridine ;

$R_1$ et $R_2$, indépendamment l'un de l'autre désignent hydrogène ou le groupement RNHCO- ;

dans laquelle R désigne alkyle en $C_1$-$C_6$, $R_1$ et $R_2$ ne désignant pas simultanément hydrogène; et

n est égal à 0 ou 1,

ainsi que leurs formes tautomères ou isomères.

Les exemples suivants sont destinés à illustrer l'invention sans pour autant présenter un caractère limitatif.

EXEMPLE 1

Synthèse du pipéridino-6 diamino-2,4 pyrimidine oxyde-3 (7)

1ère étape

Préparation de la N-(amino-2 chloro-6 pyrimidinyl-4), N'-butyl urée

A une suspension de 5 g de chloro-6 diamino-2,4 pyrimidine dans 50 $cm^3$ de DMSO on ajoute 8,6 $cm^3$ de butylisocyanate, puis on chauffe le milieu réactionnel à 80 ° C jusqu'à disparition du produit de départ en CCM.

Après hydrolyse du milieu réactionnel le produit est extrait à l'acétate d'éthyle. La phase organique est lavée, séchée puis concentrée sous pression réduite.

On obtient 5,1 g de produit qui sont purifiés par recristallisation dans l'acétate d'éthyle.

La N-(amino-2 chloro-6 pyrimidinyl-4), N'-butyl urée cristallise sous forme de cristaux blancs fondant à 227 ° C.

Le spectre $^1$H RMN 250 MHz est conforme à la structure attendue.

2ème étape

Préparation de la N-(amino-2 oxyde-3 chloro-6 pyrimidinyl-4), N'-butyl urée

A une suspension de 2,8 g de N-(amino-2 chloro-6 pyrimidinyl-4), N'-butyl urée préparée précédemment dans un mélange de 100 $cm^3$ d'alcool et 15 $cm^3$ d'eau, on ajoute 2,17 g d'acide métachloroperbenzoïque.

La température de la réaction est maintenue à 20 ° C et on suit son évolution en CCM.

Après transformation du produit de départ, l'alcool est évaporé sous pression réduite et le résidu est repris dans de l'eau additionnée d'hydrogénocarbonate de sodium.

Après une demi-heure d'agitation vigoureuse de la phase aqueuse, le précipité est filtré, lavé abondamment à l'eau et séché.

On obtient 2,5 g de cristaux blancs dont le point de fusion est de 195-196 ° C et dont le spectre $^1$H RMN est conforme à la structure attendue.

2ème étape bis

Préparation de la N-(amino-2 oxyde-3 chloro-6 pyrimidinyl-4) N'-butyl urée Oxydation à l'eau oxygénée

A une solution de 1,9 g de N-(amino-2 chloro-6 pyrimidinyl-4), N′-butyl urée, préparée précédemment dans 40 $cm^3$ de dichlorométhane et 4 $cm^3$ d'acide formique, on ajoute 1,9 $cm^3$ d'eau oxygénée. La solution est portée à 40° C pendant 4 heures. Après transformation du produit de départ, le dichlorométhane est évaporé sous pression réduite et le résidu est repris à l'eau. Le précipité est filtré, lavé abondamment à l'eau et séché. On obtient 1,4 g de cristaux correspondant au produit attendu et dont le point de fusion est de 194-195° C.

3ème étape

Préparation de la N-(pipéridino-6 amino-2 oxyde-3 pyrimidinyl-4), N′-butyl urée

Une suspension de 1 g de N-(amino-2 oxyde-3 chloro-6 pyrimidinyl-4), N′-butyl urée préparée précédemment dans 10 $cm^3$ de pipéridine est chauffée pendant 1 heure à 100° C.

Le milieu réactionnel est versé sur de l'eau glacée. Le précipité obtenu est filtré, séché et recristallisé dans le méthanol.

On obtient 0,950 mg de N-(pipéridino-6 amino-2 oxyde-3 pyrimidinyl-4), N′-butyl urée.

4ème étape

Préparation du pipéridino-6 diamino-2,4 pyrimidine oxyde-3

Une solution de 1 g de N-(pipéridino-6 amino-2 oxyde-3 pyrimidinyl-4), N′-butyl urée dans un mélange de 50 $cm^3$ d'isopropanol et 25 $cm^3$ de potasse 10 N est chauffée à 80° C jusqu'à disparition du produit de départ.

L'isopropanol est évaporé sous pression réduite et le produit attendu cristallise dans la phase aqueuse. La phase aqueuse est diluée avec 50 $cm^3$ d'eau et le produit est filtré.

Par recristallisation dans le méthanol, on obtient 550 mg de pipéridino-6 diamino-2,4 pyrimidine oxyde-3 dont le point de fusion est de 260° C.

Dans l'exemple précédent, chaque composé intermédiaire est purifié par recristallisation. Il est possible de conduire cette synthèse jusqu'à la dernière étape sans purification des intermédiaires.

On donne ci-après les quantités obtenues à chacune de ces étapes.

EXEMPLE 1 bis

1ère étape

Préparation de la N-(amino-2 chloro-6 pyrimidinyl-4), N′-butylurée.

100 g de chloro-6 diamino-2,4 pyrimidine en solution dans 1 litre de N-méthyl pyrrolidone sont traités par 95 $cm^3$ de n-butylisocyanate à 95° C pendant 9 heures. A la fin de la réaction, le mélange réactionnel est versé dans 5 litres d'eau. Le précipité obtenu est essoré et séché.

On obtient 164 g de N-(amino-2 chloro-6 pyrimidinyl-4) N′-butylurée brute contenant environ 20% de son isomère.

2ème étape

Préparation de la N-(amino-2 oxyde-3 chloro-6 pyrimidinyl-4), N′-butylurée.

A une suspension de 150 g du produit brut obtenu à l'étape précédente dans 2 litres de dioxanne et 200 $cm^3$ d'acide formique, on ajoute 175 $cm^3$ d'eau oxygénée à 110 volumes. Le mélange est porté à une température de 60° C pendant 48 heures. Après filtration sur célite, l'excès d'eau oxygénée est détruit par addition d'une solution aqueuse d'hydrogénosulfite. Puis, le dioxanne est éliminé par évaporation sous vide. Le N-oxyde obtenu à la température ambiante sous forme de cristaux est essoré, lavé à l'eau et séché.

On obtient 92 g de N-(amino-2 oxyde-3 chloro-6 pyrimidinyl-4), N′-butylurée.

Dans ce mode opératoire, la filtration sur célite s'effectue à 40° C; on élimine ainsi la majorité de l'isomère en position 2; ce dernier ne s'oxyde pas dans ces conditions.

3ème étape

Préparation de la N-(pipéridino-6 amino-2 oxyde-3 pyrimidinyl-4), N′-butylurée.

Une suspension de 90 g de N-(amino-2 oxyde-3 chloro-6 pyrimidinyl-4), N′-butylurée obtenue à l'étape précédente dans 70 $cm^3$ d'éthanol et 170 $cm^3$ de pipéridine, est portée sous agitation à 50 °C pendant 4 heures. Le milieu devient progressivement homogène. Une partie du chlorhydrate de pipéridine précipité à la température ambiante est éliminée par filtration. La solution est concentrée de façon que le volume final soit de 500 $cm^3$. Elle est versée, sous forte agitation, sur 1,5 litre d'eau glacée.

On obtient 97 g de N-(pipéridino-6 amino-2 oxyde-3 pyrimidinyl-4, N′-butylurée.

4ème étape

Préparation du pipéridino-6 diamino-2,4 pyrimidine oxyde-3.

Une solution de 90 g du produit obtenu dans l'étape précédente dans 750 $cm^3$ de butanol et 10 $cm^3$ d'eau est portée, sous agitation, à une température de 100 °C. On ajoute 20 g de potasse en pastilles en petites portions à cette solution. Le chauffage est maintenu pendant 3 heures. Après refroidissement, la solution est lavée à l'eau et le butanol est éliminé par évaporation sous pression réduite. Le produit obtenu est alors agité dans 200 $cm^3$ d'acétate d'éthyle à température ambiante; on extrait ainsi la majorité des impuretés.

On obtient 45 g de pipéridino-6 diamino-2,4 pyrimidine oxyde-3 que l'on recristallise dans un mélange acétonitrile-méthanol.

EXEMPLE 2

Synthèse du pipéridino-6 diamino-2,4 pyrimidine oxyde-3 à partir de la diamino-2,4 hydroxy-6 pyrimidine.

1ère étape

Synthèse de la N-(amino-4 hydroxy-6 pyrimidinyl-2), N′-cyclohexyl urée.

A une suspension de 10 g de diamino-2,4 hydroxy-6 pyrimidine dans environ 100 $cm^3$ de N-méthyl pyrrolidone, portée à 60 °C, on ajoute goutte à goutte une solution de 10 $cm^3$ d'isocyanate de cyclohexyle dans 50 $cm^3$ de N-méthyl pyrrolidone. A la fin de l'addition, le chauffage est maintenu pendant 3 heures. Puis on rajoute 2 $cm^3$ d'isocyanate de cyclohexyle et on porte le milieu réactionnel à 90 °C pendant 2 heures. Après vérification en chromatographie sur couche mince de la disparition totale du produit de départ, on additionne un mélange de 5 $cm^3$ d'acide acétique dans 20 $cm^3$ d'eau et on abandonne le milieu réactionnel pendant une nuit à température ambiante.

La solution est versée sur 11 d'eau et on maintient l'agitation pendant environ 1 heure. Le produit attendu est filtré et après séchage on récupère 17 g d'une poudre blanche dont le point de fusion est de 236 °C (capillaire).

| Analyse élémentaire : $C_{11}H_{17}N_5O_2$ | | | | |
|---|---|---|---|---|
| | C% | H% | N% | O% |
| Valeur calculée : | 52,57 | 6,82 | 27,87 | 12,73 |
| Valeur trouvée : | 51,85 | 6,75 | 27,53 | 12,55 |

2ème étape

Synthèse de la N-(paratolylsulfonyloxy-6 amino-4 pyrimidinyl-2), N′-cyclohexylurée.

A une suspension de 8 g de N-(amino-4 hydroxy-6 pyrimidinyl-2), N′-cyclohexylurée et de 12,2 g de chlorure de paratoluène sulfonyle dans un mélange de 120 $cm^3$ d'eau et de 40 $cm^3$ d'acétone portée à 40 °C, on ajoute goutte à goutte une solution de soude 1N. On suit la réaction en contrôlant en continu le pH du milieu réactionnel pour s'assurer que la soude additionnée est rapidement consommée.

Lorsque la réaction n'évolue plus on vérifie en chromatographie sur couche mince que tout le produit de départ a disparu et on rajoute 100 $cm^3$ de soude diluée pour éliminer l'excès de chlorure de paratoluène sulfonyle.

Le précipité ainsi obtenu est filtré et lavé abondamment à l'eau (jusqu'à neutralité des eaux de rinçage). Après séchage on récupère 8,3 g de produit pur fondant à 203-205 ° C.

| Analyse élémentaire : $C_{18}H_{23}N_5O_4S$ | | | | | |
|---|---|---|---|---|---|
| | C% | H% | N% | O% | S% |
| Valeur calculée : | 53,32 | 5,72 | 17,27 | 15,78 | 7,91 |
| Valeur trouvée : | 53,11 | 5,65 | 17,31 | 15,63 | 7,88 |

3ème étape

Synthèse de la N′-(paratolylsulfonyloxy-6 amino-4 oxyde-3 pyrimidinyl-2), N′-cyclohexylurée.

A une suspension de 3 g de N-(paratolylsulfonyloxy-6 amino-4 pyrimidinyl-2), N′-cyclohexylurée dans 100 $cm^3$ de dioxanne on ajoute 20 $cm^3$ d'acide formique et 6 $cm^3$ d'eau oxygénée à 110 volumes et on porte le milieu réactionnel à 55 ° C pendant environ 1 heure (le produit de départ passe en solution). On ajoute 3 $cm^3$ d'eau oxygénée à 110 volumes et on maintient le chauffage pendant encore 1 heure.

Le mélange réactionnel est versé sur 300 $cm^3$ d'eau glacée et le précipité qui se forme est filtré et lavé abondamment à l'eau.

Après séchage on récupère une poudre blanche dont le spectre $^1H$ RMN 80 MHz est conforme à la structure attendue et dont le point de fusion est de 215 ° C. (le produit commence à brunir à partir de 130 ° C).

4ème étape

Synthèse de la N-(pipéridino-6 amino-4 oxyde-3 pyrimidinyl-2), N′-cyclohexyurée.

A une solution de 1,5 g de N-(paratolyl sulfonyloxy-6 amino-4 pyrimidinyl-2), N′-cyclohexylurée dans 50 $cm^3$ de THF on ajoute 0,5 $cm^3$ de pipéridine. On agite le milieu réactionnel pendant une 1/2 heure à température ambiante puis on chauffe à 60 ° C pendant 1 heure. A la fin de la réaction le milieu est versé dans l'eau et extrait à l'acétate d'éthyle.

Le brut de réaction est purifié par chromatographie sur gel de silice (éluant : $CH_2Cl_2$-MeOH) et on récupère 750 mg d'une poudre blanche légèrement rosée dont le spectre $^1H$ RMN 80 MHz correspond à la structure attendue et qui fond à 183-185 ° C.

5ème étape

Préparation du pipéridino-6 diamino-2,4 pyrimidine oxyde-3.

L′ N-(pipéridino-6 amino-4 oxyde-3 pyrimidinyl-2), N′-cyclohexylurée est traitée suivant les conditions de la 4ème étape de l'exemple I.
Le temps de réaction de coupure de la fonction urée dans cette position 2 est plus long que lorsque l'urée est en position 4 (exemple I).

**Revendications**
**Revendications pour les Etats contractants suivants : AT, BE, CH, DE, FR, GB, GR, IT, LI, NL, SE**

**1.** Procédé de préparation de pipéridino-6 diamino-2,4 pyrimidine oxyde-3, caractérisé par le fait que l'on protège dans une première étape, au moins l'une des deux fonctions amine d'un composé de formule (1) :

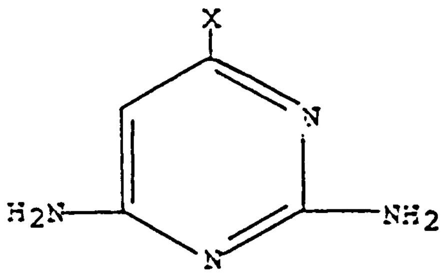

(1)

dans laquelle X désigne halogène ou un groupement OH par une réaction d'addition d'un isocyanate de formule :

R - N = C = O

dans laquelle R désigne un groupement alkyle ayant 1 à 6 atomes de carbone sur l'un des groupements amine du composé de formule (1), que l'on oxyde ensuite le dérivé d'urée résultant pour transformer le composé en N-oxyde correspondant, le N-oxyde étant ensuite amené à réagir avec la pipéridine puis soumis à une réaction d'élimination du ou des groupements protecteurs par traitement avec une base organique ou inorganique.

**2.** Procédé selon la revendication 1, caractérisé par le fait que la première étape du procédé est effectuée dans un solvant aprotique conduisant à la formation d'un composé répondant à la formule (2) :

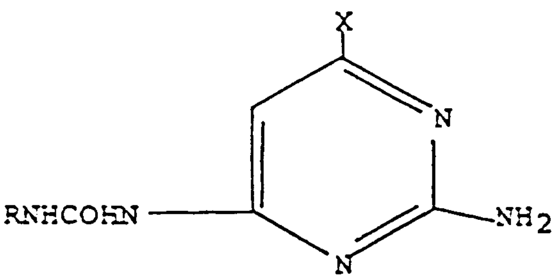

(2)

dans laquelle X et R ont les mêmes significations qu'indiquées ci-dessus.

**3.** Procédé selon la revendication 1, caractérisé par le fait que l'on utilise comme solvant du tétrahydrofuranne et qu'on obtient un composé répondant à la formule (3) :

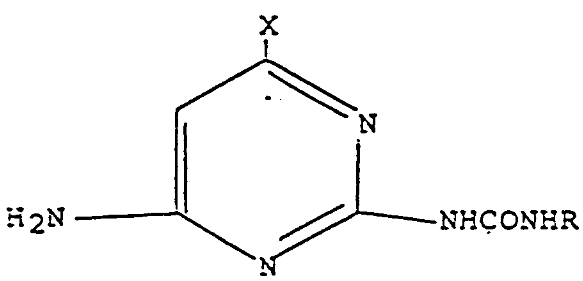

(3)

dans laquelle X et R ont les mêmes significations que celles indiquées ci-dessus.

**4.** Procédé selon la revendication 1, caractérisé par le fait que l'on utilise comme solvant de la N-méthylpyrrolidone et qu'on obtient un composé répondant à la formule (3) :

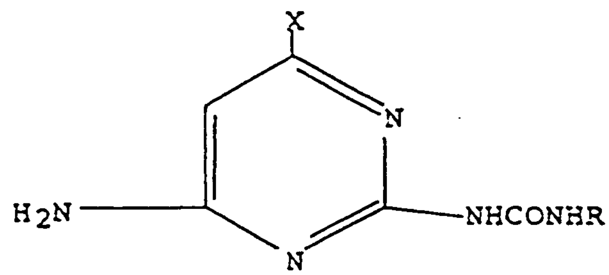

(3)

dans laquelle X est OH et R a les mêmes significations que celles indiquées ci-dessus.

5. Procédé selon l'une quelconque des revendications 1 ou 2, caractérisé par le fait que l'on procède à l'oxydation du composé de formule (2) dans lequel X désigne chlore en phase hétérogène dans un mélange d'un solvant chloré et d'acide formique ou un mélange de dioxanne et d'acide formique et d'un excès d'eau oxygénée de 2 à 5 équivalents par rapport au composé 2a.

6. Procédé selon la revendication 1, caractérisé par le fait que l'on transforme la mono-urée de formule (3) dans laquelle X désigne OH en tosylate ou en benzène sulfonate correspondant et que le composé en résultant est ensuite oxydé en N-oxyde-3.

7. Procédé selon l'une quelconque des revendications 1 à 6, caractérisé par le fait que les N-oxyde-3 sont amenés à réagir avec la pipéridine utilisée en excès.

8. Procédé selon l'une quelconque des revendications 1 à 7, caractérisé par le fait que l'élimination du ou des groupements protecteurs urée est mise en oeuvre par traitement avec une base forte, minérale ou organique.

9. Composés nouveaux, caractérisé par le fait qu'ils répondent à la formule :

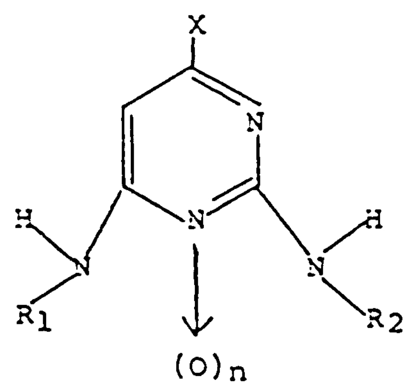

dans laquelle :

X désigne OH, Cl, O-tosyl, O-benzènesulfonyl;

$R_1$ et $R_2$, indépendamment l'un de l'autre, désignent hydrogène ou le groupement RNHCO-;

dans laquelle R désigne alkyle en $C_1$-$C_6$, $R_1$ et $R_2$ ne désignant pas simultanément hydrogène; et

n est égal à 0 ou 1,

ainsi que leurs formes tautomères ou isomères.

**Revendications pour l'Etat contractant suivant : ES**

1. Procédé de préparation de pipéridino-6 diamino-2,4 pyrimidine oxyde-3, caractérisé par le fait que l'on protège dans une première étape, au moins l'une des deux fonctions amine d'un composé de formule (1) :

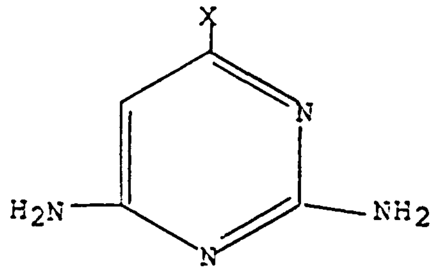

(1)

dans laquelle X désigne halogène ou un groupement OH par une réaction d'addition d'un isocyanate de formule :

R - N = C = O

dans laquelle R désigne un groupement alkyle ayant 1 à 6 atomes de carbone sur l'un des groupements amine du composé de formule (1), que l'on oxyde ensuite le dérivé d'urée résultant pour transformer le composé en N-oxyde correspondant, le N-oxyde étant ensuite amené à réagir avec la pipéridine puis soumis à une réaction d'élimination du ou des groupements protecteurs par traitement avec une base organique ou inorganique.

2. Procédé selon la revendication 1, caractérisé par le fait que la première étape du procédé est effectuée dans un solvant aprotique conduisant à la formation d'un composé répondant à la formule (2) :

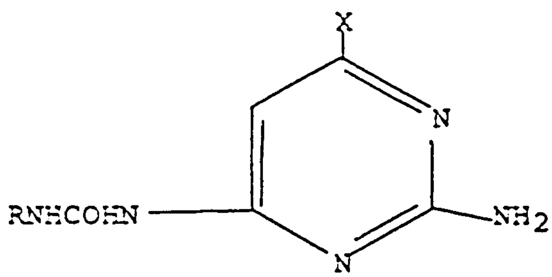

(2)

dans laquelle X et R ont les mêmes significations qu'indiquées ci-dessus.

3. Procédé selon la revendication 1, caractérisé par le fait que l'on utilise comme solvant du tétrahydrofuranne et qu'on obtient un composé répondant à la formule (3) :

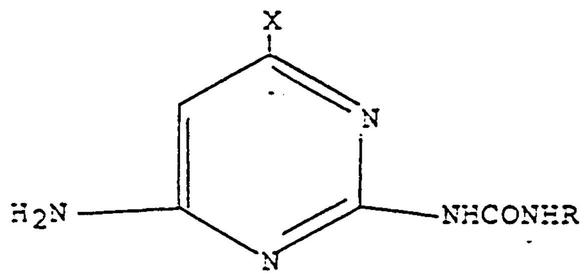

(3)

dans laquelle X et R ont les mêmes significations que celles indiquées ci-dessus.

4. Procédé selon la revendication 1, caractérisé par le fait que l'on utilise comme solvant de la N-méthylpyrrolidone et qu'on obtient un composé répondant à la formule (3) :

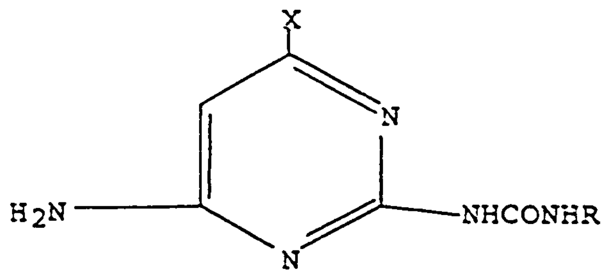

(3)

dans laquelle X est OH et R a les mêmes significations que celles indiquées ci-dessus.

**5.** Procédé selon l'une quelconque des revendications 1 ou 2, caractérisé par le fait que l'on procède à l'oxydation du composé de formule (2) dans lequel X désigne chlore en phase hétérogène dans un mélange d'un solvant chloré et d'acide formique ou un mélange de dioxanne et d'acide formique et d'un excès d'eau oxygénée de 2 à 5 équivalents par rapport au composé 2a.

**6.** Procédé selon la revendication 1, caractérisé par le fait que l'on transforme la mono-urée de formule (3) dans laquelle X désigne OH en tosylate ou en benzène sulfonate correspondant et que le composé en résultant est ensuite oxydé en N-oxyde-3.

**7.** Procédé selon l'une quelconque des revendications 1 à 6, caractérisé par le fait que les N-oxyde-3 sont amenés à réagir avec la pipéridine utilisée en excès.

**8.** Procédé selon l'une quelconque des revendications 1 à 7, caractérisé par le fait que l'élimination du ou des groupements protecteurs urée est mise en oeuvre par traitement avec une base forte, minérale ou organique.

**Claims**

**Claims for the following Contracting States : AT, BE, CH, DE, FR, GB, GR, IT, LI, NL, SE**

**1.** Process for preparing 6-piperidino-2,4-diaminopyrimidine 3-oxide, characterised in that at least one of the two amine functions of a compound of formula (1)

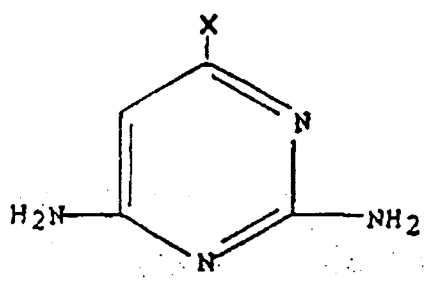

(1)

in which X denotes halogen or an OH group, is protected in a first step by an addition reaction of an isocyanate of formula:

R - N = C = O

in which R denotes an alkyl group having 1 to 6 carbon atoms, with one of the amine groups of the compound of formula (1) and in that the resulting urea derivative is then oxidised to convert the compound to the corresponding N-oxide, the N-oxide then being reacted with piperidine and thereafter subjected to a reaction of removal of the protective group or groups by treatment with an organic or inorganic base.

**2.** Process according to Claim 1, characterised in that the first step of the process is performed in an aprotic solvent, leading to the formation of a compound corresponding to the formula (2):

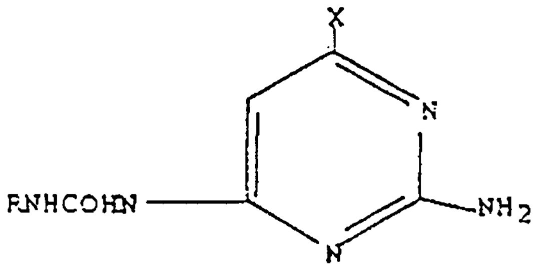

(2)

in which X and R have the same meanings as stated above.

**3.** Process according to Claim 1, characterised in that tetrahydrofuran is used as a solvent, and in that a compound corresponding to the formula (3):

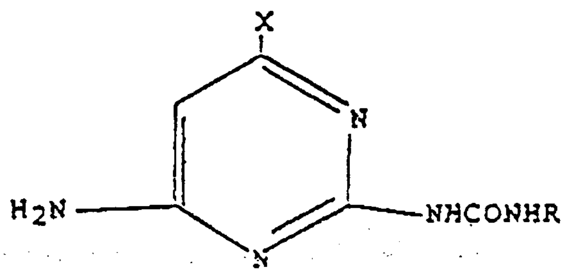

(3)

in which X and R have the same meanings as those stated above, is obtained.

**4.** Process according to Claim 1, characterised in that N-methylpyrrolidone is used as a solvent, and in that a compound corresponding to the formula (3):

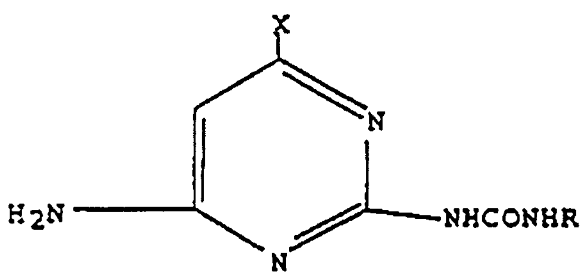

(3)

in which X is OH and R has the same meanings as those stated above, is obtained.

**5.** Process according to either of Claims 1 and 2, characterised in that the oxidation of the compound of formula (2) in which X denotes chlorine is performed in a heterogeneous phase in a mixture of a chlorinated solvent and formic acid or a mixture of dioxane and formic acid and an excess of hydrogen peroxide of 2 to 5 equivalents relative to the compound 2a.

**6.** Process according to Claim 1, characterised in that the monourea of formula (3) in which X denotes OH is converted to the corresponding tosylate or benzenesulphonate, and in that the compound resulting therefrom is then oxidized to the $N^3$-oxide.

**7.** Process according to any one of Claims 1 to 6, characterised in that the $N^3$-oxides are reacted with piperidine used in excess.

**8.** Process according to any one of Claims 1 to 7, characterised in that the removal of the protective urea group or groups is performed by treatment with a strong inorganic or organic base.

**9.** New compounds, characterised in that they correspond to the formula:

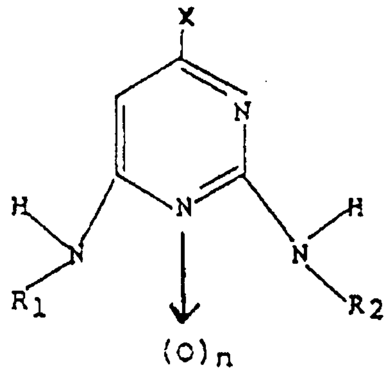

in which:

X denotes OH, Cl, O-tosyl or O-benzenesulphonyl;

$R_1$ and $R_2$, independently of one another, denote hydrogen or a group RNHCO-;

in which R denotes $C_1$-$C_6$alkyl, $R_1$ and $R_2$ not simultaneously denoting hydrogen; and

n is equal to 0 or 1,

as well as their tautomeric or isomeric forms.

**Claims for the following Contracting State : ES**

**1.** Process for preparing 6-piperidino-2,4-diaminopyrimidine 3-oxide, characterised in that at least one of the two amine functions of a compound of formula (1)

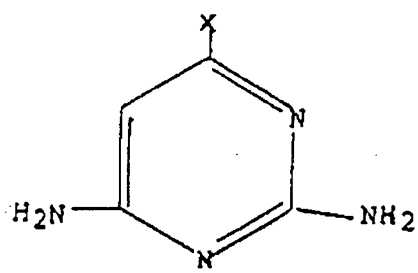

(1)

in which X denotes halogen or an OH group, is protected in a first step by an addition reaction of an isocyanate of formula:

R - N = C = O

in which R denotes an alkyl group having 1 to 6 carbon atoms, with one of the amine groups of the compound of formula (1) and in that the resulting urea derivative is then oxidised to convert the compound to the corresponding N-oxide, the N-oxide then being reacted with piperidine and thereafter subjected to a reaction of removal of the protective group or groups by treatment with an organic or inorganic base.

**2.** Process according to Claim 1, characterised in that the first step of the process is performed in an aprotic solvent, leading to the formation of a compound corresponding to the formula (2):

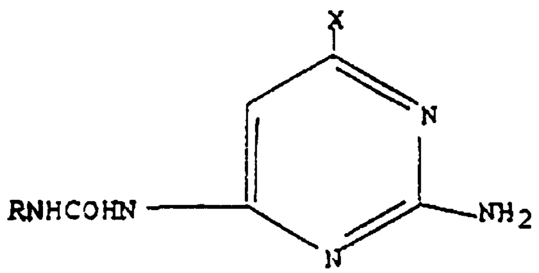

(2)

in which X and R have the same meanings as stated above.

**3.** Process according to Claim 1, characterised in that tetrahydrofuran is used as a solvent, and in that a compound corresponding to the formula (3):

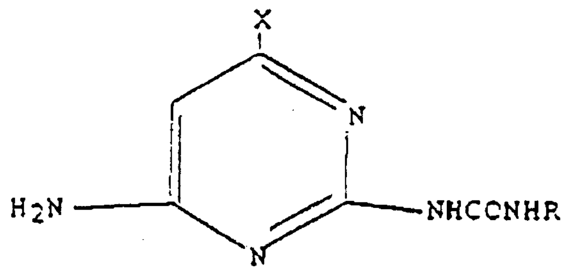

(3)

in which X and R have the same meanings as those stated above, is obtained.

**4.** Process according to Claim 1, characterised in that N-methylpyrrolidone is used as a solvent, and in that a compound corresponding to the formula (3):

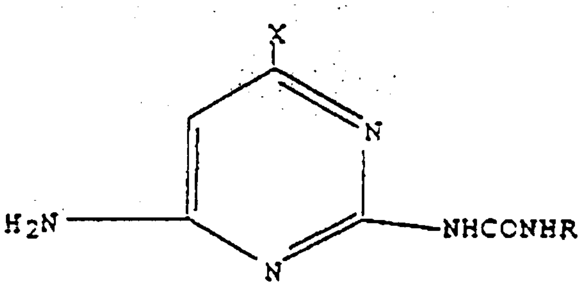

(3)

in which X is OH and R has the same meanings as those stated above, is obtained.

**5.** Process according to either of Claims 1 and 2, characterised in that the oxidation of the compound of formula (2) in which X denotes chlorine is performed in a heterogeneous phase in a mixture of a chlorinated solvent and formic acid or a mixture of dioxane and formic acid and an excess of hydrogen peroxide of 2 to 5 equivalents relative to the compound 2a.

**6.** Process according to Claim 1, characterised in that the monourea of formula (3) in which X denotes OH is converted to the corresponding tosylate or benzenesulphonate, and in that the compound resulting therefrom is then oxidized to the $N^3$-oxide.

**7.** Process according to any one of Claims 1 to 6, characterised in that the $N^3$-oxides are reacted with piperidine used in excess.

**8.** Process according to any one of Claims 1 to 7, characterised in that the removal of the protective urea group or groups is performed by treatment with a strong inorganic or organic base.

**Patentansprüche**

**Patentansprüche für folgende Vertragsstaaten : AT, BE, CH, FR, DE, GB, GR, IT, LI, NL, SE**

1. Verfahren zur Herstellung von 6-Piperidino-2,4-diaminopyrimidin-3-oxid,
dadurch **gekennzeichnet,** daß man
in einer ersten Stufe mindestens eine der Aminfunktionen der Verbindung der Formel (1):

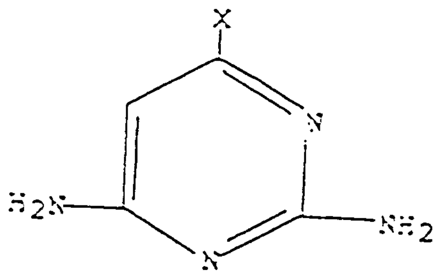

(1)

worin X Halogen oder eine OH-Gruppe bedeutet, durch eine Additionsreaktion eines Isocyanats der Formel:

R - N = C = O

worin R eine Alkylgruppe mit 1 bis 6 Kohlenstoffatomen bedeutet, an eine der Amingruppen der Verbindung der Formel (1) schützt und das sich ergebende Harnstoffderivat oxidiert, um die Verbindung in das entsprechende N-Oxid zu überführen, worauf man das N-Oxid mit Piperidin reagieren läßt und dann die Schutzgruppe(n) durch Behandlung mit einer organischen oder anorganischen Base eliminiert.

2. Verfahren gemäß Anspruch 1,
dadurch **gekennzeichnet,** daß
man die erste Stufe des Verfahrens in einem aprotischen Lösungsmittel durchführt, wobei eine Verbindung der Formel (2) gebildet wird:

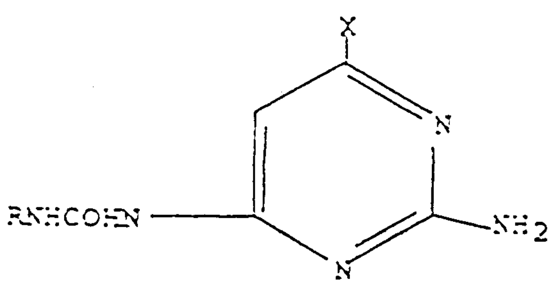

(2)

worin X und R dieselben Bedeutungen wie oben angegeben haben.

3. Verfahren gemäß Anspruch 1,
dadurch **gekennzeichnet,** daß
man als Lösungsmittel Tetrahydrofuran verwendet und eine Verbindung der Formel (3) erhält:

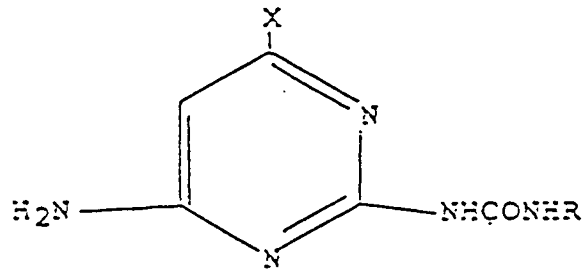

(3)

worin X und R dieselben Bedeutungen wie oben angegeben haben.

**4.** Verfahren gemäß Anspruch 1,
dadurch **gekennzeichnet,** daß
man als Lösungsmittel N-Methylpyrrolidon verwendet und eine Verbindung der Formel (3) erhält:

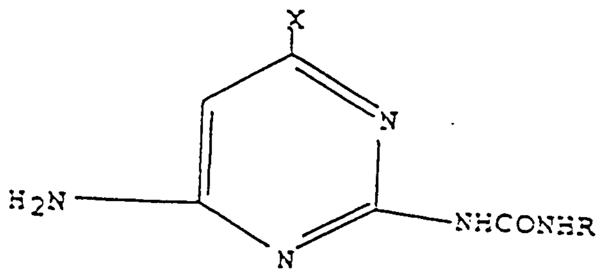

(3)

worin X OH ist und R dieselbe Bedeutung wie oben angegeben hat.

**5.** Verfahren gemäß jedem der Ansprüche 1 oder 2,
dadurch **gekennzeichnet,** daß
man die Verbindung der Formel (2), worin X Chlor bedeutet, in heterogener Phase in einer Mischung aus einem chlorierten Lösungsmittel und Ameisensäure oder einer Mischung aus Dioxan und Ameisensäure und mit einem Überschuß eines mit Sauerstoff beladenen Wassers von 2 bis 5 'quivalenten, bezogen auf Verbindung 2a, oxidiert.

**6.** Verfahren gemäß Anspruch 1,
dadurch **gekennzeichnet,** daß
man den Monoharnstoff der Formel (3), worin X OH bedeutet, in das entsprechende Tosylat oder Benzolsulfonat überführt und die dabei sich ergebende Verbindung dann zum 3-N-Oxid oxidiert.

**7.** Verfahren gemäß jedem der Ansprüche 1 bis 6,
dadurch **gekennzeichnet,** daß
man die 3-N-Oxide mit im Überschuß angewandtem Piperidin reagieren läßt.

**8.** Verfahren gemäß jedem der Ansprüche 1 bis 7,
dadurch **gekennzeichnet,** daß
man die Harnstoffschutzgruppe(n) durch Behandlung einer starken mineralischen oder organischen Base eliminiert.

**9.** Neue Verbindungen,
dadurch **gekennzeichnet,** daß
sie durch die Formel dargestellt sind:

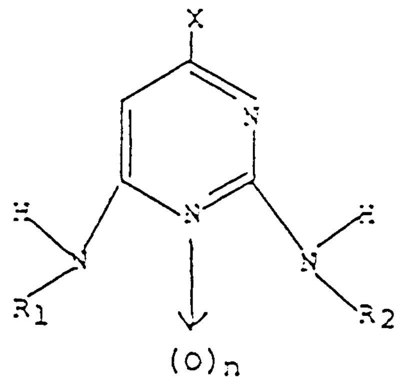

worin:
X OH, Cl, O-Tosyl, O-Benzolsulfonyl bedeutet,
$R_1$ und $R_2$, unabhängig voneinander, Wasserstoff oder die RNHCO-Gruppe bedeuten,
worin R $C_1$-$C_6$-Alkyl bedeutet, und wobei $R_1$ und $R_2$ nicht gleichzeitig Wasserstoff darstellen, und worin n gleich 0 oder 1 ist,
sowie deren tautomere oder isomere Formen.

**Patentansprüche für folgenden Vertragsstaat : ES**

**1.** Verfahren zur Herstellung von 6-Piperidino-2,4-diaminopyrimidin-3-oxid,
dadurch **gekennzeichnet,** daß man
in einer ersten Stufe mindestens eine der Aminfunktionen der Verbindung der Formel (1):

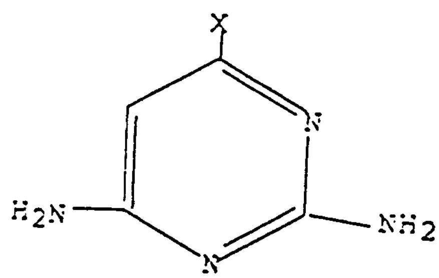

(1)

worin X Halogen oder eine OH-Gruppe bedeutet, durch eine Additionsreaktion eines Isocyanats der Formel:

R - N = C = O

worin R eine Alkylgruppe mit 1 bis 6 Kohlenstoffatomen bedeutet, an eine der Amingruppen der Verbindung der Formel (1) schützt und das sich ergebende Harnstoffderivat oxidiert, um die Verbindung in das entsprechende N-Oxid zu überführen, worauf man das N-Oxid mit Piperidin reagieren läßt und dann die Schutzgruppe(n) durch Behandlung mit einer organischen oder anorganischen Base eliminiert.

**2.** Verfahren gemäß Anspruch 1,
dadurch **gekennzeichnet,** daß
man die erste Stufe des Verfahrens in einem aprotischen Lösungsmittel durchführt, wobei eine Verbindung der Formel (2) gebildet wird:

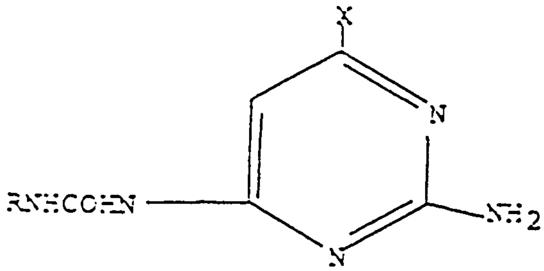

(2)

worin X und R dieselben Bedeutungen wie oben angegeben haben.

3. Verfahren gemäß Anspruch 1, dadurch **gekennzeichnet,** daß man als Lösungsmittel Tetrahydrofuran verwendet und eine Verbindung der Formel (3) erhält:

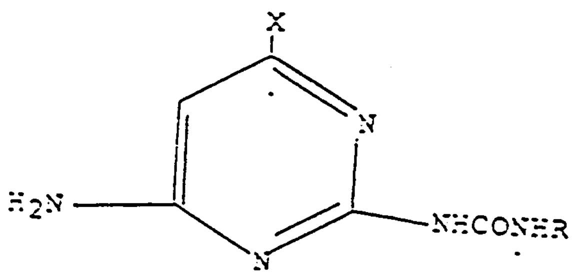

(3)

worin X und R dieselben Bedeutungen wie oben angegeben haben.

4. Verfahren gemäß Anspruch 1, dadurch **gekennzeichnet,** daß man als Lösungsmittel N-Methylpyrrolidon verwendet und eine Verbindung der Formel (3) erhält:

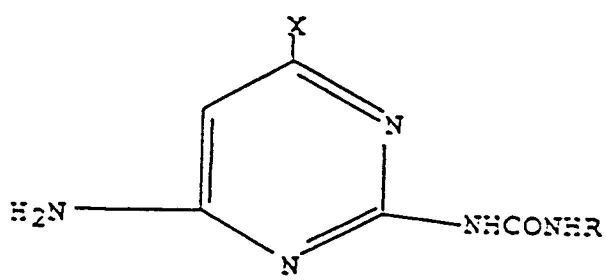

(3)

worin X OH ist und R dieselbe Bedeutung wie oben angegeben hat.

5. Verfahren gemäß jedem der Ansprüche 1 oder 2, dadurch **gekennzeichnet,** daß man die Verbindung der Formel (2), worin X Chlor bedeutet, in heterogener Phase in einer Mischung aus einem chlorierten Lösungsmittel und Ameisensäure oder einer Mischung aus Dioxan und Ameisensäure und mit einem Überschuß eines mit Sauerstoff beladenen Wassers von 2 bis 5 Äquivalenten, bezogen auf Verbindung 2a, oxidiert.

6. Verfahren gemäß Anspruch 1, dadurch **gekennzeichnet,** daß man den Monoharnstoff der Formel (3), worin X OH bedeutet, in das entsprechende Tosylat oder Benzolsulfonat überführt und die dabei sich ergebende Verbindung dann zum 3-N-Oxid oxidiert.

7. Verfahren gemäß jedem der Ansprüche 1 bis 6,
dadurch **gekennzeichnet,** daß
man die 3-N-Oxide mit im Überschuß angewandtem Piperidin reagieren läßt.

8. Verfahren gemäß jedem der Ansprüche 1 bis 7,
dadurch **gekennzeichnet,** daß
man die Harnstoffschutzgruppe(n) durch Behandlung mit einer starken mineralischen oder organischen Base eliminiert.